(19) **Europäisches Patentamt / European Patent Office / Office européen des brevets**

(11) **EP 3 014 265 B1**

(12) # FASCICULE DE BREVET EUROPEEN

(45) Date de publication et mention de la délivrance du brevet:
**21.11.2018 Bulletin 2018/47**

(21) Numéro de dépôt: **14742273.7**

(22) Date de dépôt: **25.06.2014**

(51) Int Cl.:
*A23L 17/60* [(2016.01)]  *A23C 9/13* [(2006.01)]
*G01N 30/72* [(2006.01)]  *G01N 33/00* [(2006.01)]
*G01N 33/04* [(2006.01)]  *G01N 33/10* [(2006.01)]

(86) Numéro de dépôt international:
**PCT/FR2014/051589**

(87) Numéro de publication internationale:
**WO 2014/207377 (31.12.2014 Gazette 2014/53)**

(54) **COMPOSITIONS DE FARINE DE MICROALGUES DE QUALITE SENSORIELLE OPTIMISEE**

MIKROALGENMEHLZUSAMMENSETZUNGEN MIT OPTIMIERTER SENSORISCHER QUALITÄT

MICROALGAL FLOUR COMPOSITIONS OF OPTIMISED SENSORY QUALITY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.06.2013 FR 1356113**

(43) Date de publication de la demande:
**04.05.2016 Bulletin 2016/18**

(73) Titulaire: **Corbion Biotech, Inc.**
**South San Francisco, CA 94080 (US)**

(72) Inventeurs:
• **DRUON, Amandine**
  **F-59000 Lille (FR)**
• **JEROSCH, Heike**
  **F-59940 Estaires (FR)**
• **GUILLEMANT, Maryline**
  **F-62120 Aire Sur La Lys (FR)**
• **PATINIER, Samuel**
  **F-59890 Quesnoy-Sur-Deûle (FR)**

(74) Mandataire: **Cabinet Becker et Associés**
**25, rue Louis le Grand**
**75002 Paris (FR)**

(56) Documents cités:
**US-A1- 2010 303 989**

• **J.L GUIL-GUERRERO ET AL: "Functional properties of the biomass of three microalgal species", JOURNAL OF FOOD ENGINEERING, vol. 65, no. 4, 1 décembre 2004 (2004-12-01), pages 511-517, XP055134285, ISSN: 0260-8774, DOI: 10.1016/j.jfoodeng.2004.02.014**
• **CALLEJON R M ET AL: "Volatile and sensory profile of organic red wines produced by different selected autochthonous and commercial Saccharomyces cerevisiae strains", ANALYTICA CHIMICA ACTA, ELSEVIER, AMSTERDAM, NL, vol. 660, no. 1-2, 15 février 2010 (2010-02-15), pages 68-75, XP026867216, ISSN: 0003-2670 [extrait le 2009-10-01]**
• **Jaclyn E R Santos ET AL: "Analysis of Volatile Organic Compounds in Virgin Coconut Oil and their Sensory Attibutes", Philippine Journal of Science, 19 décembre 2011 (2011-12-19), pages 161-171, XP055139768, Extrait de l'Internet: URL:http://philjournalsci.dost.gov.ph/vol1 40no2/pdfs/Analysis of volatile organic compounds in VCO.pdf [extrait le 2014-09-11]**

**Description**

La présente invention est relative à de nouvelles compositions de farine de microalgues du genre *Chlorella* présentant un profil sensoriel optimisé, ce qui permet de les incorporer dans des formulations alimentaires sans génération d'arômes indésirables, ainsi qu'une méthode pour évaluer le profil organoleptique d'une composition de farine de microalgues du genre *Chlorella.*

*Présentation de l'état de l'art*

Ne nécessitant historiquement « que de l'eau et de la lumière du soleil » pour croître, les algues sont considérées depuis longtemps comme une source de nourriture.

Il existe plusieurs espèces d'algues pouvant être utilisées en alimentation, la plupart étant des "macro-algues" comme le varech, la laitue de mer (*Ulva lactuca*) et des algues rouges de type *Porphyra* (cultivée au Japon) ou « dulse » (*Palmaria palmata*).

Cependant, outre ces macroalgues, il y a aussi d'autres sources d'algues représentées par les « microalgues », c'est à dire des algues microscopiques unicellulaires photosynthétiques ou non, d'origine marine ou non, cultivées pour leurs applications dans les biocarburants ou l'alimentation.

Par exemple, la spiruline (*Arthrospira platensis*) est cultivée dans des lagunes ouvertes (par phototrophie) pour une utilisation comme complément alimentaire ou incorporée en petites quantités dans la confiserie ou les boissons (généralement moins de 0,5% poids / poids).

D'autres microalgues riches en lipides, y compris certaines espèces de type *Chlorella,* sont aussi très populaires dans les pays asiatiques comme compléments alimentaires (mention est faite de microalgues du genre *Crypthecodinium* ou *Schizochytrium* producteurs d'acides gras omega 3).

La production et l'utilisation de la farine de microalgues de type *Chlorella* sont par exemple décrits dans les documents WO 2010/120923 et WO 2010/045368.

La fraction huile de la farine de microalgues, qui peut être composée essentiellement d'huiles mono-insaturées, peut offrir des avantages nutritionnels et santé par rapport aux huiles saturées, hydrogénées et polyinsaturés souvent trouvées dans les produits alimentaires conventionnels.

Lorsque l'on souhaite fabriquer industriellement des poudres de farine de microalgues à partir de leur biomasse, d'importantes difficultés demeurent, non seulement du point de vue technologique, mais également du point de vue du profil sensoriel des compositions produites.

En effet, si des poudres d'algues par exemple fabriquées avec des algues cultivées photosynthétiquement dans des étangs extérieurs ou par photobioréacteurs sont disponibles dans le commerce, elles ont une couleur vert foncée (liée à la chlorophylle) et un goût fort, désagréable.

Même formulées dans des produits alimentaires ou comme compléments nutritionnels, ces poudres d'algues communiquent toujours cette couleur verte visuellement peu attrayante au produit alimentaire ou au complément nutritionnel et ont un goût désagréable de poisson ou la saveur d'algues marines.

Par ailleurs, il est connu que certaines espèces d'algues bleues produisent naturellement des molécules chimiques odorantes telles que la géosmine (trans-1,10-diméthyle-trans-9-decalol) ou le MIB (2-méthylisoborneol), générant des odeurs terreuses ou de moisi.

Quant aux chlorelles, le descripteur communément admis dans ce domaine est le goût de « thé vert », un peu semblable à d'autres poudres végétales vertes telles que l'orge vert en poudre ou le blé vert en poudre, goût attribué à sa forte teneur en chlorophylle.

Leur saveur n'est habituellement masquée que lorsqu'elles sont mélangées avec des légumes à saveur forte ou des jus d'agrumes.

Callejon et al (2010, Analytica Chimia Acta, 660, 68-75) décrit une méthode pour déterminer la qualité organoleptique de vins rouges bio comprenant la détermination de la teneur en composés organiques volatils.

Il existe donc toujours un besoin non satisfait de disposer de compositions de farine de microalgues du genre *Chlorella* de qualité organoleptique convenable permettant l'utilisation de celles-ci dans des produits alimentaires plus nombreux et diversifiés.

*Résumé de l'invention*

La société Demanderesse a trouvé que l'on pouvait répondre à ce besoin en proposant des compositions de farine de microalgues présentant un profil sensoriel optimisé, caractérisées par leur teneur totale en 13 composés organiques volatils.

L'invention est définie par l'objet des revendications.

Ainsi, la présente description est relative à une méthode pour déterminer la qualité organoleptique d'une composition de farine de microalgues comprenant la détermination de la teneur totale en 13 composés organiques volatils, les 13 composés organiques volatils étant l'heptanal, le 3-octène-2-one, le 2,4-heptadiénal, la 3,5-octadiène-2-one, le 2,4-nonadiénal, le 2,4-décadiénal, l'acide hexanoïque, l'acide 2-éthylhexanoïque, l'acide heptanoïque, le myristate-1, le laurate-1, le myristate-2 et la géranyl-acétone.

De préférence, la teneur totale en 13 composés organiques volatils est déterminée par SPME / GC, de préférence par SPME / GC-MS.

Ainsi, une faible teneur totale en 13 composés organiques volatils est associée à une qualité organoleptique optimisée. A contrario, une teneur totale plus élevée en 13 composés organiques volatils est associée à une qualité organoleptique moyenne, voire mauvaise ou inacceptable.

De préférence, la teneur totale en 13 composés organiques volatils est déterminée par la surface totale des pics de chromatographie après SPME / GC correspondant aux 13 composés organiques volatiles. La teneur totale en 13 composés organiques volatils, en particulier la surface totale des pics de chromatographie correspondant aux 13 composés organiques volatiles, est comparée à celle de composition(s) de farine de microalgues de référence pour laquelle ou lesquelles les qualités organoleptiques sont définies, notamment comme inacceptable ou acceptable.

La présente description est également relative à une composition de farine de microalgues présentant une qualité organoleptique optimisée, caractérisée en ce que sa teneur totale en 13 composés organiques volatils est faible, les 13 composés organiques volatils étant l'heptanal, le 3-octène-2-one, le 2,4-heptadiénal, la 3,5-octadiène-2-one, le 2,4-nonadiénal, le 2,4-décadiénal, l'acide hexanoïque, l'acide 2-éthylhexanoïque, l'acide heptanoïque, le myristate-1, le laurate-1, le myristate-2 et la géranyl-acétone. La composition est caractérisée en ce que la surface totale des pics de chromatographie après SPME / GC correspondant aux 13 composés organiques volatiles est comprise entre 1 et 25 % par rapport à celle d'une composition de farine de microalgues de référence de qualité organoleptique inacceptable.

La présente description est en outre relative à une composition de dégustation d'une composition de farine de microalgues, comprenant :

- 5-10 % de composition de farine de microalgues, de préférence environ 7 % ;
- 0,5-2% de sucre, de préférence environ 1% ;
- 0,1-0,5 % d'arôme vanille, de préférence environ 0,25% ; et
- le reste en lait écrémé, de préférence environ 91,75%.

les pourcentages étant exprimés en poids de la composition.

Ainsi, la présente description est relative à une méthode de préparation d'une composition de dégustation de composition de farine de microalgues comprenant la préparation d'une composition de dégustation telle que décrite ci-dessus, son homogénéisation, et le chauffage de la composition à 60-85°C, de préférence environ 75°C, pendant 2-10 minutes, de préférence environ 5 minutes.

Elle concerne une méthode pour tester les qualités organoleptiques d'une composition de farine de microalgues comprenant la préparation d'une composition de dégustation telle que décrite dans le présent document et l'évaluation des qualités organoleptiques par un panel de testeurs.

Enfin, la présente description est relative à un procédé non revendiqué de définition d'un profil analytique en composés volatils permettant d'évaluer la qualité organoleptique des compositions de farine de microalgues, comprenant :

- la construction d'une première matrice associant des compositions de farine de microalgues dont deux témoins de qualité organoleptique acceptable et inacceptable à l'évaluation de leurs qualités organoleptiques par un panel sensoriel d'au moins 10 personnes,
- la construction d'une seconde matrice associant à ces mêmes compositions leur caractérisation par un profil d'analyse des composés organiques volatils, et
- la corrélation de la première matrice à la seconde pour produire un modèle de relation à partir duquel les compositions présentant un profil organoleptique optimisé peuvent être ainsi caractérisées par leur profil analytique en composés organiques volatils.

De préférence, les descripteurs de l'analyse sensorielle comprennent la couleur, la texture nappante, la saveur sucrée, et les flaveurs suivantes : champignon, céréales, beurre/produit laitier, huile rance, et arrière-goût végétal.

De préférence, l'analyse sensorielle est réalisée en utilisation une composition de dégustation préparée par la méthode décrite ci-dessus et détaillée dans le présent document.

De préférence, l'analyse des composés organiques volatils est réalisée par SPME / GC-MS.

De préférence, les composés organiques volatils appartiennent aux familles des aldéhydes saturés et di-insaturés, des cétones insaturés, des acides carboxyliques et leurs dérivés.

*Description détaillée*

Au sens de l'invention, une composition de farine de microalgues présente un « profil sensoriel optimisé » ou une « qualité organoleptique optimisée », lorsque son évaluation par un panel sensoriel en formulation alimentaire (par exemple crème glacée) conclut à l'absence de défauts qui altèrent la qualité organoleptique desdites formulations alimentaires contenant ces compositions de farine de microalgues.

Par « qualité organoleptique » est entendue la propriété d'un aliment en termes de goût, odeur, aspect, couleur et consistance.

Ces défauts (terme anglo-saxon de « off-notes ») sont associés à la présence de molécules spécifiques odorantes et/ou aromatiques indésirables qui se caractérisent par un seuil de perception correspondant à la valeur minimale du stimulus sensoriel nécessaire à l'éveil d'une sensation.

Le « profil sensoriel optimisé » ou « qualité organoleptique optimisé » est alors traduit par un panel sensoriel par l'obtention des meilleurs scores sur une échelle d'évaluation des 4 critères sensoriels (aspect, texture, saveurs et flaveurs).

Par « teneur totale » est entendue la somme des teneurs pour chacun des composés organiques volatils de la liste.

Par « environ » est entendue la valeur plus ou moins 10 % de celle-ci, de préférence plus ou moins 5 % de celle-ci. Par exemple, « environ 100 » signifie entre 90 et 110, de préférence entre 95 et 105.

La société Demanderesse a découvert que le profil sensoriel d'une composition de farine de microalgues peut être également défini par la nature et le seuil de détection de molécules spécifiques odorantes, en particulier de composés organiques volatils particuliers. En effet, elle a identifié un ensemble de 13 composés organiques volatils dont la teneur globale dans une composition de farine de microalgues permet de déterminer la qualité organoleptique de celle-ci. Ces 13 composés organiques volatils sont les suivants : l'heptanal, le 3-octène-2-one, le 2,4-heptadiénal, la 3,5-octadiène-2-one, le 2,4-nonadiénal, le 2,4-décadiénal, l'acide hexanoïque, l'acide 2-éthylhexanoïque, l'acide heptanoïque, le myristate-1, le laurate-1, le myristate-2 et la géranyl-acétone.

Ainsi, la présente description est relative à une méthode pour déterminer la qualité organoleptique d'une composition de farine de microalgues comprenant la détermination de la teneur totale en 13 composés organiques volatils, les 13 composés organiques volatils étant l'heptanal, le 3-octène-2-one, le 2,4-heptadiénal, la 3,5-octadiène-2-one, le 2,4-nonadiénal, le 2,4-décadiénal, l'acide hexanoïque, l'acide 2-éthylhexanoïque, l'acide heptanoïque, le myristate-1, le laurate-1, le myristate-2 et la géranyl-acétone.

La méthode n'exclut pas la détermination de la teneur d'autres composés organiques volatils. Cependant, les 13 composés organiques volatils sont suffisants pour déterminer la qualité organoleptique d'une composition de farine de microalgues.

De préférence, ces composés organiques volatils sont prélevés par micro extraction en phase solide (SPME) et analysés par chromatographie en phase gazeuse GC, en particulier par GC-MS (chromatographie en phase gazeuse couplée à la spectrométrie de masse).

La fraction volatile est extraite de l'échantillon de la composition de farine de microalgues en chauffant celle-ci pendant une durée suffisante en présence d'une fibre de SPME. La fibre peut être par exemple choisie, de manière non-exhaustive, parmi le groupe consistant en carboxen et polydiméthylsiloxane (CAR/PDMS), divinylbenzène, Carboxen et polydiméthylsiloxane (DVB/CAR/PDMS), un alliage de métal et de polydiméthylsiloxane (PDMS), une fibre Carbopack-Z® (carbone noir graphitisé), polyacrylate, Carbowax® polyéthylèneglycol (PEG), et PDMS / DVB. De préférence, on utilise une fibre DVB/CAR/PDMS.

Par exemple, l'extraction peut se faire à une température comprise entre 40 et 70°C, de préférence entre 50 et 65°C, en particulier environ 60 °C pendant au moins 10 minutes, de préférence au moins 15 minutes et par exemple entre 15 minutes et 1 heure.

De préférence, cette étape d'extraction est faite dans un récipient scellé. Une quantité suffisante d'échantillon doit être utilisée, par exemple au moins 1 g, notamment entre 1 et 10 g et en particulier environ 3 g. La technique de SPME est bien connue de l'homme du métier et fait partie de ces connaissances générales.

Les composés organiques volatils sont ensuite désorbés, à une température compatible avec le type de fibre SPME utilisée, par exemple entre 250 et 270 °C pour la fibre utilisée dans nos essais, plus précisément à 250°C, et injectés dans le système d'analyse.

De préférence, l'analyse est faite par chromatographie en phase gazeuse GC, en particulier par GC-MS.

Plusieurs dispositifs de GC/MS sont disponibles commercialement, par exemple le spectromètre GC/Masse Clarus (PerkinElmer, USA), le chromatographe en phase gazeuse Hewlett Packard 6890 (Hewlett Packard, USA) et le chromatographe en phase gazeuse Aglient 6890N couplé détecteur sélectif de masse Aglient 5973. Les méthodes d'ionisation qui peuvent être utilisées en GC/MS sont par exemple la spectroscopie de masse avec ionisation en mode impact électronique (EI), en mode impact chimique (CI) l'ionisation par électronébulisateur, la désorption/ionisation laser assistée par matrice (MALDI), la décharge luminescente, la désorption par champs (FD), etc.

Les colonnes utilisées pour la GC sont de préférence une colonne Cp-Wax 52CB 60m * 0.32mm ; df 0.25μm ou équivalent que nous avons retenu ; autre colonne testée était une de type ZB-1ms 30m * 0.25mm ; df 1μm.

Ainsi, la hauteur ou la surface du pic de chromatographie correspondant au composé organique volatil est corrélée à la quantité dudit composé. Par « surface du pic » est entendue la surface d'un ion spécifique sous la courbe dans le chromatogramme SPME-GC/MS.

De préférence, la teneur en un des 13 composés organiques volatils est déterminée par la surface du pic de l'ion spécifique du chromatogramme SPME-GC/MS correspondant à ce composé organique volatile.

En outre, la teneur totale en 13 composés organiques volatils est de préférence déterminée par la surface totale des pics de chromatographie après SPME / GC correspondant aux 13 composés organiques volatiles.

La teneur en composés organiques volatils est déterminée, notamment en comparaison à celle de produit de référence. De préférence, on considère la teneur totale en composés organiques volatils de la liste des 13 définie dans la présente description.

Ainsi, une faible teneur totale en 13 composés organiques volatils est associée à une qualité organoleptique optimisée. A contrario, une teneur totale plus élevée en 13 composés organiques volatils est associée à une qualité organoleptique moyenne, voire mauvaise ou inacceptable.

Dans un premier aspect, le produit de référence est un produit dans lequel la quantité en un composé organique volatil est connue.

Idéalement, le produite de référence pourrait comprendre les 13 composés organiques volatils de la liste en des quantités prédéfinies. Un tel produit de référence permet de déterminer exactement la quantité des composés organiques volatils.

Dans un deuxième aspect, le produit de référence est une composition de farine de microalgues dont le profil organoleptique est défini. Le profil organoleptique est de préférence déterminé par la méthode détaillée plus loin dans le présent document ou dans la partie expérimentale. Ainsi, ce produit de référence permet de déterminer des quantités relatives en composés organiques volatils.

Dans cet aspect, lorsque la composition de farine de microalgues de référence présente un profil organoleptique acceptable ou tout juste acceptable, la teneur en composés organiques volatils, de préférence la teneur totale en lesdits 13 composés organiques volatils, est comparée en celle déterminée pour le produit de référence. Si elle est supérieure ou égale à celle du produit de référence, alors la composition testée est considérée comme ayant une qualité organoleptique insuffisante ou inacceptable. Si elle est inférieure, alors la composition testée est considérée comme ayant une qualité organoleptique acceptable ou optimisée.

Dans cet aspect, lorsque la composition de farine de microalgues de référence présente un profil organoleptique inacceptable, voire très inacceptable, la teneur en composés organiques volatils, de préférence la teneur totale en lesdits 13 composés organiques volatils, est comparée en celle déterminée pour le produit de référence. Si elle est égale ou supérieure à celle du produit de référence, alors la composition testée est considérée comme ayant une qualité organoleptique insuffisante ou inacceptable. Si elle est inférieure, alors la composition testée est considérée comme ayant une qualité organoleptique acceptable ou optimisée. Notamment, il a été défini dans les exemples en utilisant une composition de farine de microalgues de référence présentant un profil organoleptique très inacceptable, que la composition testée a un profil organoleptique optimisé lorsque la teneur totale en lesdits 13 composés organiques volatils est comprise entre 1 et 25 % par rapport à celle de cette composition de référence (qui est considérée comme étant 100 %). Par exemple, la teneur totale d'une composition d'une qualité organoleptique acceptable est au minimum deux fois inférieure à celle d'une composition d'une qualité organoleptique inacceptable, par exemple au minimum 2, 3, ou 4 fois inférieure, et dans un aspect le plus exigeant, au minimum 10 fois inférieure.

Dans un aspect préféré, est considéré dans la méthode la teneur en lesdits 13 composés organiques volatils, en particulier leur teneur totale, et ce après SPME/GC-EI/MS.

La méthode permet ainsi de classer les compositions de farine de microalgues en fonction de leur qualité organoleptique, et de qualifier les formulations alimentaires les contenant, par la quantification desdits composés organiques volatils particuliers.

La présente description est également relative à une composition de farine de microalgues présentant une qualité organoleptique optimisée, caractérisée en ce que sa teneur totale en 13 composés organiques volatils est faible, les 13 composés organiques volatils étant l'heptanal, le 3-octène-2-one, le 2,4-heptadiénal, la 3,5-octadiène-2-one, le 2,4-nonadiénal, le 2,4-décadiénal, l'acide hexanoïque, l'acide 2-éthylhexanoïque, l'acide heptanoïque, le myristate-1, le laurate-1, le myristate-2 et la géranyl-acétone.

En effet, il a été déterminé par les travaux de la société Demanderesse que, plus la teneur totale en 13 composés organiques volatils est faible, et plus la qualité organoleptique de la composition de farine de microalgues est bonne. A contrario, plus la teneur totale en 13 composés organiques volatils est élevée, et plus la qualité organoleptique de la composition de farine de microalgues est mauvaise.

La teneur en composés organiques volatils est déterminée comme détaillé ci-dessus dans la méthode d'évaluation de la qualité organoleptique d'une composition de farine de microalgues. Cette teneur est évaluée par rapport à celle de produit de référence comme défini ci-dessus.

Par exemple, la teneur totale en lesdits composés organiques volatils est faible en comparaison d'une composition de farine de microalgues présentant une qualité organoleptique inacceptable, voire très inacceptable. Par exemple, la teneur totale d'une composition d'une qualité organoleptique acceptable est au minimum deux fois inférieure à celle d'une composition d'une qualité organoleptique inacceptable, par exemple au minimum 2, 3, ou 4 fois inférieure, et dans un aspect le plus exigeant, au minimum 10 fois inférieure. De préférence, la composition est caractérisée en ce que la surface totale des pics de chromatographie après SPME / GC correspondant aux 13 composés organiques volatiles est comprise entre 1 et 25 % par rapport à celle d'une composition de farine de microalgues de référence de qualité organoleptique inacceptable.

Par « composition de farine de microalgues » est entendu une composition comprenant au minimum 50, 60, 70, 80 ou 90 % en poids sec de biomasse de microalgues. Cependant, d'autres ingrédients peuvent facultativement être compris dans cette composition.

Au sens de la présente description, le terme « farine de microalgues » doit être compris dans son interprétation la plus large et comme désignant par exemple, une composition comprenant une pluralité de particules de biomasse de microalgues. La biomasse de microalgues est dérivée de cellules de microalgues, qui peuvent être entières ou cassées, ou un mélange de cellules entières et cassées.

Un certain nombre de documents de l'état de l'art, comme la demande de brevet internationale WO 2010/120923, décrivent des méthodes de fabrication et d'utilisation en alimentation de la biomasse de microalgues de *Chlorella.*

Les microalgues dont il est question dans la présente description sont donc des microalgues du genre *Chlorella,* plus particulièrement *Chlorella protothecoides,* plus particulièrement encore des *Chlorella* privées de pigments chlorophylliens, par toute méthode connue en soi de l'homme du métier (soit par le fait que la culture est réalisée à l'obscurité, soit parce que la souche a été mutée de manière à ne plus produire ces pigments). Notamment, les microalgues peuvent être choisies, de manière non-exhaustive, parmi les *Chlorella protothecoides, Chlorella kessleri, Chlorella minutissima, Chlorella sp., Chlorella sorokiniama, Chlorella luteoviridis, Chlorella vulgaris*, *Chlorella reisiglii, Chlorella ellipsoidea, Chlorella saccarophila*, *Parachlorella kessleri, Parachlorella beijerinkii, Prototheca stagnora* et *Prototheca moriformis.Ainsi,* dans un aspect tout particulier, la composition de farine de microalgues est une composition de farine de *Chlorella,* et en particulier de *Chlorella protothecoides.*

Le procédé fermentaire décrit dans cette demande de brevet WO 2010/120923 permet ainsi la production d'un certain nombre de compositions de farine de microalgues de qualité sensorielle variable. La méthode telle que décrite dans le présent document permet donc de sélectionner les compositions de farine de microalgues présentant un profil organoleptique acceptable, notamment pour les applications alimentaires, sans pour cela devoir organiser des évaluations organoleptiques par un panel de personnes.

La présente description est également relative à une composition de dégustation de compositions de farine de microalgues. En effet, la société Demanderesse a défini une matrice de dégustation très simple. Pourtant, elle permet de faire une évaluation organoleptique similaire à celle obtenue avec des recettes beaucoup plus complexes et très différentes telles qu'une crème glacée ou une brioche. L'évaluation avec cette matrice de dégustation est beaucoup plus précise ou exacte que celle faite avec une simple solution aqueuse, qui s'est révélée être incapable de prédire les qualités organoleptiques des compositions de farine de microalgues dans une glace, par exemple.

Par conséquence, la présente description concerne une composition de dégustation de compositions de farine de microalgues comprenant :

- 5-10 % de composition de farine de microalgues, de préférence environ 7 % ;
- 0,5-2% de sucre, de préférence environ 1% ;
- 0,1-0,5 % d'arôme vanille, de préférence environ 0,25% ; et
- le reste en lait écrémé, de préférence environ 91,75%.

les pourcentages étant exprimés en poids de la composition.

Par ailleurs, la présente description est relative à une méthode de préparation de la composition de dégustation de composition de farine de microalgues comprenant la préparation d'une composition de dégustation telle que décrite ci-dessus, son homogénéisation, et le chauffage de la composition à 60-85°C, de préférence environ 75°C, pendant 2-10 minutes, de préférence environ 5 minutes.

Elle concerne en outre une méthode pour tester les qualités organoleptiques d'une composition de farine de microalgues comprenant la préparation d'une composition de dégustation telle que décrite ci-dessus et l'évaluation des qualités organoleptiques par un panel de testeurs. Cette évaluation peut notamment être réalisée par les méthodes détaillées ci-dessous.

La société Demanderesse propose également un procédé de définition d'un profil analytique en composés volatils permettant d'évaluer la qualité organoleptique des compositions de farine de microalgues, comprenant :

- la construction d'une première matrice associant des compositions de farine de microalgues dont, de préférence, deux témoins de qualité organoleptique acceptable et inacceptable à l'évaluation de leurs qualités organoleptiques par un panel sensoriel d'au moins 10 personnes,
- la construction d'une seconde matrice associant à ces mêmes compositions leur caractérisation par un profil d'analyse des composés organiques volatils, et
- la corrélation de la première matrice à la seconde pour produire un modèle de relation à partir duquel les compositions présentant un profil organoleptique optimisé peuvent être ainsi caractérisées par leur profil analytique en composés organiques volatils.

Un panel sensoriel est formé pour évaluer les propriétés sensorielles de différents lots de compositions de farine de microalgues, en particulier de farine de biomasse de *Chlorella protothecoides.*

Un ensemble de personnes, au minimum 10, 20 ou 30, en particulier environ 15, est rassemblé pour évaluer des descripteurs de plusieurs compositions de farine de microalgues, de préférence en comparaison d'un échantillon de farine de microalgues de référence identifié conforme, c'est-à-dire de qualité organoleptique acceptable, (lot référence n°1) et un autre échantillon de qualité organoleptique très inacceptable (lot référencé n°2).

De préférence, les compositions de farine de microalgues sont testées sous la forme d'une composition de dégustation selon le présent document. Alternativement, les compositions peuvent être testées sous toute autre forme désirée par l'homme du métier, par exemple sous forme de crème glacée ou de produit de panification comme une brioche.

De préférence, les produits de référence tels que présentés dans le tableau suivant sont associés à chaque descripteur :

| Descripteurs | | Référence |
|---|---|---|
| Aspect | Couleur (de clair à foncé) | |
| Texture | Nappant | lait entier + 5% crème |
| Saveurs | Sucré | Saccharose 1% |
| Flaveurs | Champignon | 100 g de champignons dans 100 ml d'eau froide / dilution X 4 |
| | Céréales | Solution Ebly 10% |
| | Beurre / produit laitier | |
| | Huile rance | Huile oxydée 1,5% |
| | Arrière goût végétal | Composition de farine de microalgues très inacceptable |

Bien entendu, l'homme du métier peut définir d'autres produits de référence s'il le souhaite.

A chaque séance de dégustation, plusieurs produits, par exemple 4 à 5, sont évalués sur chaque descripteur en comparaison d'un lot ou échantillon de référence, de préférence de référence considéré comme de qualité organoleptique acceptable.

Tous les produits sont évalués les uns après les autres, sur des échelles allant par exemple de 1 à 9 de la manière suivante :

Valeur de 1 : le descripteur évalué n'est pas présent dans le produit ;

Valeur de 5 : le descripteur évalué est présent dans le produit exactement de la même façon que sur le produit de référence de qualité organoleptique acceptable ;

Valeur de 9 : le descripteur évalué est très présent dans le produit.

Le lot de référence de qualité organoleptique acceptable est une composition de farine de microalgues conforme au sens où elle présente le profil sensoriel "satisfaisant" tous ces descripteurs. Le lot de référence de qualité organoleptique très inacceptable est un lot ne satisfaisant pas les descripteurs relatifs aux notes aromatiques, c'est-à-dire aux descripteurs Saveurs et Flaveurs, car il présente un arrière goût végétal important.

Il est important de noter que le lot de référence de qualité organoleptique acceptable n'est pas forcément la composition de farine de microalgues présentant le profil sensoriel optimal : il s'agit de préférence d'une composition de farine de microalgues perçue par le panel sensoriel comme "satisfaisant", notamment présentant une note de 5 sur tous les descripteurs testés.

Dans cet aspect, les autres compositions de farine de microalgues, les compositions testées, sont classées par le panel sensoriel de part et autre de ce lot de référence de qualité organoleptique acceptable.

De manière générale, les autres compositions testées sont classées par le panel sensoriel par rapport au(x) lot(s) de référence de qualité organoleptique acceptable ou inacceptable, de préférence acceptable.

Ainsi, la première étape aboutit au classement des différentes compositions de farine de microalgues testées en fonction de leur qualité organoleptique.

Notamment, des analyses de variances (ANOVA) sont réalisées pour évaluer le pouvoir discriminant des descripteurs (descripteurs dont la p-value associée au test de Fisher - ANOVA de type 3 - est inférieure à 0,20 pour l'effet Composition dans le modèle descripteur ~ Composition + juge). L'effet Composition s'interprète comme le pouvoir discriminant des descripteurs : s'il n'y a pas d'effet (Probabilité Critique > 0,20), les compositions n'ont pas été discriminées selon ce critère. Plus la probabilité critique est petite, plus le descripteur est discriminant. Une Analyse en Composantes Principales (ACP) est ensuite réalisée afin d'obtenir une cartographie sensorielle des compositions, et une représentation simultanée de toutes les compositions sur tous les descripteurs.

Ce classement sert alors ensuite de base pour étudier le profil d'analyse des composés organiques volatils et sélectionner des molécules responsables de la mauvaise qualité organoleptique des compositions de farine de microalgues.

Ainsi, le profil des composés organiques volatils des compositions de farine de microalgues est déterminé. Il est déterminé par toute méthode connue de l'homme du métier, et de préférence par SPME / GC-MS, tel que détaillé ci-dessus.

Dans un exemple très spécifique, pour l'analyse en SPME / GC-MS des différents lots de compositions de farine de microalgues, on procède comme suit.

Une prise d'essai de 3 g d'échantillon est introduite dans un flacon SPME (20 ml) scellé et incubée à 60°C pendant 15 min puis extraite à 60°C pendant 45 min avec une fibre SPME en DVB/CAR/PDMS (abréviation anglo-saxonne pour divinylbenzene / carboxen / polydimethylsiloxane, df 50/30 $\mu$m).

Les composés organiques volatils extraits sont désorbés à 250°C dans l'injecteur du système GC-MS TSQ de Thermo Scientific, injectés en mode « splitless »puis séparés sur une colonne CPwax52 (60m * 0.25 mm, 0.25 $\mu$m) avec le gaz Helium à 1.5 ml/min. Le programme de température est : 50°C isotherme pendant 3 min, puis programmation à 5°C/minute jusque 230°C puis isotherme pendant 20 min. La détection est faite par spectrométrie de masse en mode impact électronique (EI) et les composés sont identifiés par comparaison avec les spectres EI de la bibliothèque NIST.

L'analyse des composés volatils donne des chromatogrammes en GC-MS très complexes, avec un très grand nombre de pics. Par des analyses de variances et des régressions linéaires, les composés organiques volatils qui sont le mieux corrélés aux résultats obtenus pour la matrice sensorielle et aux « off-notes » sont sélectionnés. De préférence, les composés organiques volatils appartiennent à 4 familles de composés : les aldéhydes (saturés et insaturés), les cétones insaturés, les acides carboxyliques et les dérivés d'acides carboxyliques.

Ainsi, un profil organoleptique optimisé est associé et caractérisé par un profil analytique en composés organiques volatils.

Dans un aspect préféré, les différents composés organiques sélectionnés seront considérés par leur teneur totale, en comparaison à des compositions de référence, notamment telles que définies précédemment. En particulier, la surface totale des pics de chromatographie correspondant aux composés organiques volatils sélectionnés sera considérée et comparée.

**EXEMPLES**

**Exemple 1. Définition du test sensoriel**

_Test sensoriel classiquement mis en oeuvre_

La perception d'une composition de farine de microalgue est généralement déterminée par solubilisation dans de l'eau, milieu neutre par excellence.

Un panel sensoriel a donc été formé pour évaluer les propriétés sensorielles de différents lots de farine de microalgues, préparées selon l'enseignement de la demande de brevet WO 2010/12093.

Huit lots de farine de microalgues ont été testés : lot 21, lot 23, lot 24, lot 31, lot 53, lot 61, lot 111 et lot 131 en regard d'un échantillon de référence identifié conforme ou acceptable (lot référence n°1) et un autre lot très inacceptable (lot référencé n°2).

Un ensemble de 14 personnes a été rassemblé pour générer des descripteurs autour d'une composition de farine de microalgue considéré comme conforme (lot référencé 1), composition placée en solution aqueuse à 10% à température ambiante (jusqu'à homogénéisation).

La liste des descripteurs retenus est la suivante :

- Aspect : Couleur

- Texture : Nappant

- Saveurs : Sucré, Amer

- Arômes : champignon, céréale, beurre / produits laitiers, huile rance

Le Tableau 1 suivant présente les produits de référence associés par le panel à chaque descripteur :

Tableau 1.

| Descripteurs | | Référence |
|---|---|---|
| Aspect | Couleur (de clair à foncé) | |
| Texture | Nappant | lait entier + 5% crème |
| Saveurs | Sucré | Saccharose 1% |
| | Amertume | Quinine 0,0075 g/l |
| Flaveurs | Champignon | 100 g de champignons dans 100 ml d'eau froide / dilution X 4 |
| | Céréales | Solution de blé de la marque EBLY 10% |
| | Beurre / produit laitier | |
| | Huile rance | Huile oxydée 1,5% |

A chaque séance de dégustation, 4 à 5 compositions sont évaluées sur chaque descripteur en comparaison à une composition identifiée comme acceptable (lot référence 1).

Toutes les compositions sont évaluées les unes après les autres, sur des échelles allant de 1 à 9.

1 : le descripteur évalué n'est pas présent dans le produit

5 : le descripteur évalué est présent dans le produit exactement de la même façon que sur le produit de référence 1

9 : le descripteur évalué est très présent dans le produit

Par exemple, le descripteur « sucré » est évalué comme suit :

Référence

| 1 | 2 | 3 | 4 | 5 | 6 | 7 | 8 | 9 |
|---|---|---|---|---|---|---|---|---|

Le tableau 2 suivant présente le résultat des analyses sensorielles effectuées par le Panel sur 6 différents lots de compositions de farine de microalgues de qualité organoleptique variable :

Tableau 2

| | Test sensorial en solution |
|---|---|
| **Lot référence 1** | Acceptable |
| **Lot 24** | Acceptable |
| **Lot 53** | Acceptable |
| **Lot 61** | Inacceptable |

(suite)

|  | Test sensorial en solution |
|---|---|
| **Lot 111** | Acceptable |
| **Lot 131** | Acceptable |

Il apparait que 5 des 6 lots testés présentent une qualité organoleptique semblable au témoin, ce qui semblerait indiquer que leur procédé de fabrication est suffisamment maîtrisé pour conduire à l'obtention de compositions de qualité organoleptique « acceptable ».

Des essais en applications crèmes glacées ont été réalisés ensuite afin de vérifier la pertinence du classement des compositions de farine de microalgues selon le test précédent.

La formulation de la crème glacée est la suivante :

| INGREDIENTS | Quantité (%) |
|---|---|
| Lait écrémé | 64,42 |
| Saccharose | 13,5 |
| Créme fraîche (36% fat) | 10,5 |
| Lots de Composition d'algues | 4 |
| Lait écrémé en poudre | 3,5 |
| ROCLYS® 4080 commercialisé ROQUETTE FRERES | 3 |
| Stabilisant (CREMODAN® SE30) | 0,55 |
| Arôme vanille IFF 10836706 | 0,5 |
| Sel | 0,03 |
| Total (%) | 100 |

On entreprend un test triangulaire (tel que décrit dans la norme NF V09-013 de juillet 1983) en formant un panel sensoriel constitué de 21 personnes à qui on demande de se prononcer sur la qualité organoleptique de deux lots de crème glacée fabriquée à partir de :

　　∘ le lot de référence 1 et

　　∘ un lot choisi dans la gamme dont l'appréciation dans le test sensoriel précédent (solubilisation dans l'eau) était jugé acceptable : ici le lot 24.

L'une des 2 crèmes glacées est présentée en double aux panélistes.

Il leur est ensuite demandé s'ils perçoivent une différence entre ces trois crèmes glacées.

Or 14 personnes sur 21 ont réussi à percevoir une différence ; le panel a donc perçu, contre toute attente, dans le contexte de la crème glacée, une différence organoleptique entre le lot de référence et le lot testé pourtant jugé acceptable dans le test sensoriel de la poudre solubilisée dans l'eau.

La différence est significative entre les deux compositions (distribution binomiale B (21, 1/3), avec un seuil de significativité de 0,05). Par ailleurs, 7 personnes ont également perçu un fort arrière-goût pour décrire ce lot de production.

Des essais ont alors été entrepris sur un certain nombre de lots produits.

Le tableau 3 suivant résume les résultats obtenus.

Tableau 3

|  | Test sensorial en solution | En application crème glacée |
|---|---|---|
| Lot référence 1 | acceptable | acceptable |
| Lot 24 | acceptable | inacceptable |
| Lot 53 | acceptable | inacceptable |

(suite)

|  | Test sensorial en solution | En application crème glacée |
|---|---|---|
| Lot 61 | inacceptable | inacceptable |
| Lot 111 | acceptable | acceptable |
| Lot 131 | acceptable | acceptable |

On en déduit que le test classique de mesure de la qualité organoleptique d'une composition de farine de microalgues dans de l'eau n'est pas adapté à la prédiction de la qualité organoleptique des crèmes glacées correspondantes.

Des résultats similaires ont été obtenus dans le cas de brioches préparées à partir de ces mêmes compositions de farine de microalgues.

*Test développé par la société Demanderesse pour évaluer des différentes compositions de farine de microalgues*

Etant donné ce qui précède, la société Demanderesse a choisi de développer son propre test en remplaçant la matrice de dégustation complexe utilisée pour la formulation de la crème glacée en un mélange plus simple à réaliser que la crème glacée mais plus discriminant que la solution à 10 % dans de l'eau.

La recette formulée est la suivante :

- 7% de composition de farine de microalgues
- 1% sucre cristal
- 0.25% arôme vanille ménager
- 91.75% lait écrémé.

Le mélange est homogénéisé avec un mixeur à immersion jusqu'à obtention d'un mélange homogène (environ 20 secondes) puis il est chauffé à 75°C pendant 5 minutes au bain marie.

Le descripteur amer a été enlevé car il n'était plus cohérent dans cette nouvelle matrice et un nouveau descripteur a été ajouté :

| Descripteurs | | Référence |
|---|---|---|
| Flaveurs | Arrière-goût végétal | Composition de farine de microalgues identifiée très inacceptable (lot référence 2) |

La méthode d'évaluation reste la même (utilisation d'échelles, référence à 5).

*Logiciel de traitement des données*

Les analyses ont été réalisées à l'aide du logiciel R (en vente libre) :

R version 2.14.1 (2011-12-22)
Copyright (C) 2011 The R Foundation for Statistical Computing
ISBN 3-900051-07-0
Platform: i386-pc-mingw32/i386 (32-bit)

Le logiciel est un environnement de travail qui nécessite le chargement de modules contenant les fonctions de calcul.

Les modules utilisés dans cette étude sont les suivants :

- Pour l'ACP : Package *FactoMineR* version 1.19
- Pour l'ANOVA : Package *car* version 2.0-12
- Pour la Régression Linéaire : Package *stats* version 2.14.1

*Traitement des données :*

Des analyses de variances (ANOVA) sont réalisées pour évaluer le pouvoir discriminant des descripteurs (descripteurs dont la p-value associée au test de Fisher - ANOVA de type 1 - est inférieure à 0,20 pour l'effet Composition dans le modèle *descripteur ~ composition + juge*).

L'effet « composition » s'interprète comme le pouvoir discriminant des descripteurs : s'il n'y a pas d'effet (Probabilité Critique > 0,20), les compositions n'ont pas été discriminées selon ce critère. Plus la probabilité critique est petite, plus le descripteur est discriminant.

Une Analyse en Composantes Principales (ACP) est ensuite réalisée afin d'obtenir une cartographie sensorielle des compositions, ainsi qu'une représentation simultanée de toutes les compositions sur tous les descripteurs.

Différents lots (lot 21, lot 23, lot 24, lot 31, lot 53, lot 61, lot 111 et lot 131) ont été analysés selon la méthode décrite ci-dessus.

On présente ici deux exemples sur les descripteurs "beurre / produits laitiers" et "arrière-goût végétal".

"Arrière-goût végétal"

Tableau d'analyse de variance

| | Df | Somme Sq | Moyenne Sq | F value | **Pr(>F)** |
|---|---|---|---|---|---|
| **Composition** | 9 | 109,693 | 12.1881 | 18,2423 | **< 2e-16** |
| Juge | 13 | 18,732 | 1,4409 | 2,1566 | 0,01298 |
| Résidus | 185 | 123,603 | 0,6681 | --- | |

"Beurre / produits laitiers"

Tableau d'analyse de variance

| | Df | Somme Sq | Moyenne Sq | F value | **Pr(>F)** |
|---|---|---|---|---|---|
| **Composition** | 9 | 8,292 | 0,92131 | 1,4530 | **0.1699** |
| Juge | 13 | 8,235 | 0,63347 | 0,9991 | 0,4547 |
| Résidus | 160 | 101,451 | 0,63407 | --- | |

Il apparait que les probabilités critiques associées à l'effet composition pour les 2 descripteurs étudiés sont inférieures à 0,2 : les 2 descripteurs sont donc discriminants. La probabilité critique est plus petite sur le descripteur « arrière-goût végétal » que sur le descripteur « beurre / produits laitiers » ce qui signifie qu'on observe plus de différence entre les compositions sur le premier critère que sur le deuxième.

Voici un tableau récapitulant les probabilités critiques obtenues pour les effets composition et juge pour tous les descripteurs.

| | Composition | juge |
|---|---|---|
| Couleur | 1,62E-31 | 1,16E-05 |
| arrière-goût végétal | 1,60E-21 | 1,30E-02 |
| Goût huile rance | 4,00E-06 | 9,00E-04 |
| Nappant | 1,48E-05 | 1,63E-02 |
| Céréales | 4,05E-04 | 1,94E-07 |
| Champignons | 1,37E-03 | 5,66E-05 |
| Sucré | 3,23E-03 | 4,02E-04 |
| Produits laitiers | 1,70E-01 | 4,55E-01 |

Tous les descripteurs sont discriminants, on les garde tous pour établir l'ACP.

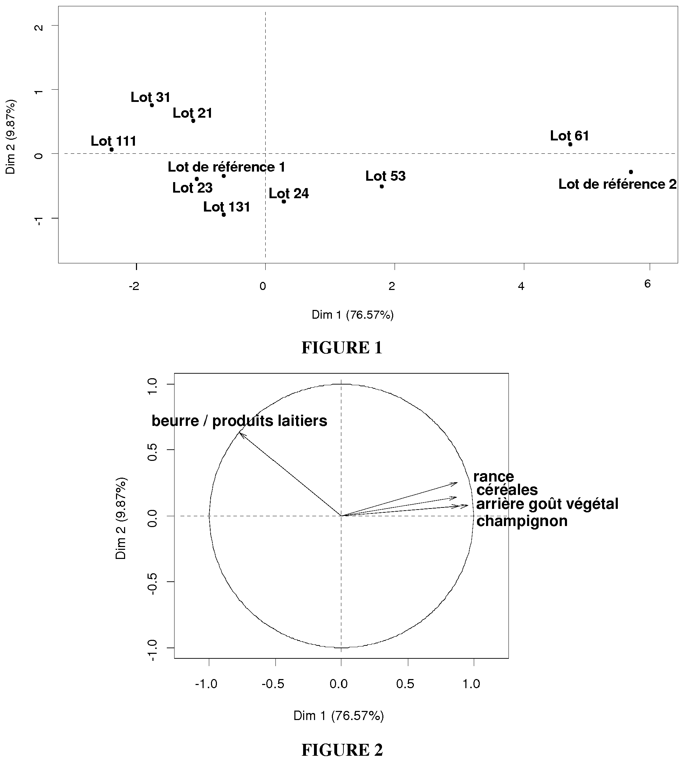

Puisque l'aromatique est un critère essentiel des compositions, l'ACP a été réalisée sur les descripteurs relatifs aux arômes uniquement (champignon, céréales, arrière-goût végétal, produit laitier, rance). La représentation graphique de cette ACP est fournie dans les Figures 1 et 2.

Le premier axe de l'ACP résumant plus de 75% de l'information (Figure 1), ce sont les coordonnées des produits sur cet axe qui nous servent de « variable / classement ». Ce classement rend donc bien compte des distances sensorielles entre les produits.

Cette méthode permet d'établir un classement de la qualité organoleptique de différentes compositions de farine de microalgues, que l'on peut figurer comme suit : Lot 111 > lot 31 > lot 21 > lot 23 > Lot référence 1 > lot 131 > lot 24 > lot 53 > lot 61 > lot référence 2.

Il existe une séparation nette entre les lots 111, 31, 21, 23 et 131 d'une part, et les lots 24, 53, et 61 d'autre part.

D'un point de vue global, le panel a jugé les lots 111, 31, 21, 23 et 131 acceptables et les lots 24, 53, et 61 inacceptable.

Cette classification organoleptique désormais établie, il est possible, efficacement selon l'invention, d'analyser le profil en SPME / GC-MS de ces échantillons afin d'identifier les cibles moléculaires de référence qui permettront de définir la qualité des compositions fabriquées.

**Exemple 2. Identification des composés organiques volatils (VOC) par SPME / GC-MS liés aux classifications organoleptiques inacceptables « off-notes »**

Pour réaliser l'analyse en SPME / GC-MS des différents lots de compositions de farine de microalgues, on procède comme suit.

Une prise d'essai de 3 g d'échantillon est introduite dans un flacon SPME (20 ml) scellé et incubée à 60°C pendant 15 min puis extraite à 60°C pendant 45 min avec une fibre SPME en DVB/CAR/PDMS (abréviation anglo-saxonne pour divinylbenzene / carboxen / polydimethylsiloxane, df 50/30 $\mu$m).

Les composés organiques volatils extraits sont désorbés dans l'injecteur du système GC-MS TSQ de Thermo Scientific, puis séparés sur une colonne CPwax52 (60m * 0.25 mm, 0.25 $\mu$m) avec le gaz Helium à 1.5 ml/min.

Le programme de température est : 50°C isotherme pendant 3 min, puis programmation à 5°C/minute jusque 230°C puis isotherme pendant 20 min.

La détection est faite par spectrométrie de masse en mode impact électronique (EI) et les composés sont identifiés par comparaison avec les spectres EI de la bibliothèque NIST.

L'analyse des composés volatils par SPME / GC-MS donne des chromatogrammes complexes, avec un très grand nombre de molécules.

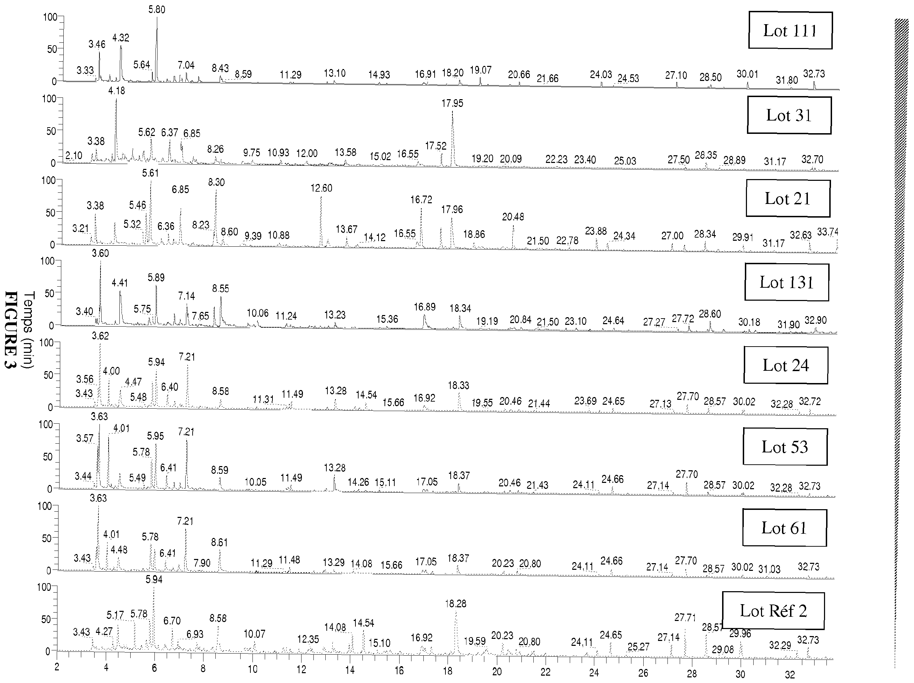

Une première approche consiste en la comparaison des profils chromatographiques (Figure 3) et déterminer si ces résultats relativement "bruts" permettent de faire le lien avec le classement sensoriel tel que présenté dans l'exemple 1.

Visuellement, les profils chromatographiques des composés volatils des différents échantillons ne reflètent pas de manière simple le classement sensoriel.

Par contre l'intégration des surfaces des pics chromatographiques entre 3,2 et 33,5 min permet d'y parvenir.

Cependant, l'erreur de l'analyse SPME / GC-MS est importante (écart type d'environ 30 %) ; il n'est donc pas aisé de différencier les échantillons classés "bons" des échantillons classés " mauvais ".

*1ère approche*

Comme la totalité des composés volatils ne reflète pas assez finement le classement sensoriel, il est décidé dans un premier temps d'identifier et de sélectionner deux molécules volatiles présentes dans l'espace de tête des échantillons susceptibles d'être responsables des descripteurs sensoriels : le nonanal et le 2,4-décadiénal qui sont des composés de dégradation des lipides bien connus pour leur impact sensoriel.

Il est possible d'introduire une notion de "valeur d'arômes" (VA) en prenant en compte le seuil olfactif connu de ces composés, selon la formule suivante :

$$VA_x = Concentration\ du\ composé\ x\ /\ seuil\ olfactif\ du\ composé\ x.$$

avec 0,07 ppb pour le seuil olfactif dans l'eau du trans-2-trans-4-décadiénal, et de 1 ppb pour le nonanal.

La VA totale correspond alors à la somme des VA individuelles des composés x pris en compte.

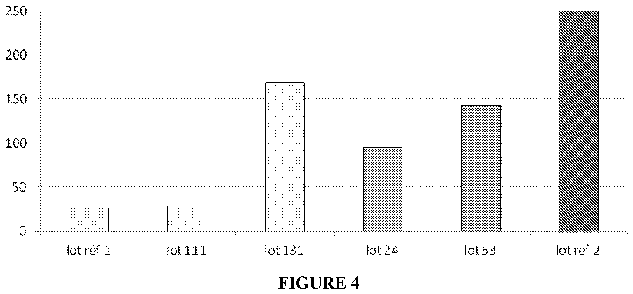

En première analyse, ce modèle semble cohérent avec l'évaluation sensorielle des différents échantillons, mais malheureusement pas de tous (Figure 4).

Il n'est donc pas possible de corréler le profil sensoriel mesuré pour chaque lot en regard de la valeur d'arôme des deux composés choisis arbitrairement.

*2ème approche*

Une seconde approche a consisté à compléter la liste des composés organiques volatils du modèle précédent en listant les composés volatils identifiés sur les chromatogrammes SPME / GC-MS qui semblent accompagner les classifications organoleptiques inacceptables « off-notes », au total 35 : Pentanal, diacétyl, hexanal, 3-pentène-2-one, heptanal, 2-pentylfurane, acetone, acétol, 2-heptenal, 3-octène-2-one, nonanal, acide acétique, 2-éthyl-1-hexanol, 2,4-heptadiénal, 3,5-octadiène-2-one, benzaldéhyde, 2-nonénal, acide butanoïque, acide isovalérique, 2,4-nonadiénal, 5,6-dihydro-2H-pyran-2-one, acide pentanoïque, 2,4-décadiénal, acide hexanoïque, géranyl-acétone, benzylalcool, phényl-léthylalcool, acide 2-éthylhexanoïque, acide heptanoïque, myristate-1, acide octanoïque, triacétine, acide nonanoïque, laurate-1, et myristate-2.

En revanche, pour les modèles suivants, les seuils olfactifs dans l'eau de certaines molécules non connus de l'homme du métier ont été définis par la société Demanderesse.

Pour sélectionner les composés organiques volatils représentatifs, on réalise une série d'analyses de la variance pour ne garder que les composés organiques volatils qui diffèrent effectivement d'une composition à l'autre compte tenu de la variabilité de la mesure SPME - GC/MS.

Le modèle est le suivant : *Composé organique volatil ~ Composition* ; on ne conserve que les composés pour lesquels la probabilité critique associée au test de Fisher est inférieure à 0,05.

On présente ici 2 exemples d'ANOVA sur les composés acide acétique et 2-éthyl-1-hexanol :

*Tableau Anova (Test de Type III)*

**Acide acétique**

|  | Somme Sq | Df | F value | **Pr(>F)** |
|---|---|---|---|---|
| *(ordonnée à l'origine)* | 5,3107e+18 | 1 | 56,504 | 0,0842. |
| **Composition** | 2,7508e+18 | 9 | 3,252 | **0,4073** |
| *Résidus* | 9,3988e+16 | 1 |  |  |

---

*Codes de significativité:* 0 '***' 0,001 '**' 0,01 '*'0,05 '.' 0,1 '' 1

*Tableau Anova (Test de Type III)*

**2-éthyl-1-hexanol**

|  | Somme Sq | Df | F value | **Pr(>F)** |
|---|---|---|---|---|
| *(Ordonnée à l'origine)* | 2,8608e+16 | 1 | 2990,30 | 0,01164 * |
| **Composition** | 2,0894e+16 | 9 | 242,67 | **0,04978** * |
| *Résidus* | 9.5669e+12 | 1 |  |  |

---

*Codes de significativité:* 0 '***' 0,001 '**' 0,01 '*'0,05 '.' 0,1 '' 1

Il apparait sur le premier composé organique volatil (l'acide acétique) que l'effet composition n'est pas significatif (probabilité critique = 0,40), ce qui signifie qu'il n'y pas de différence significative entre les produits compte tenu de la variabilité de la mesure.

Sur le 2ème composé (2-éthyl-1-hexanol), l'effet composition est bien significatif (probabilité critique < 0,05).

On gardera donc pour l'étude le 2 éthyle-1-hexanol mais pas l'acide acétique.

Après cette première sélection de composés volatils, des modèles de régressions linéaires sont établis : il s'agit d'expliquer la variable « classement sensoriel » par chaque composé un à un.

On construit donc autant de modèle qu'il y a de composés. Le modèle est le suivant : *Classement ~ Composé.*

Afin de sélectionner la liste finale de composés identifiés comme responsables des classifications organoleptiques inacceptables off-notes observées, on ne gardera que les composés pour lesquels la probabilité critique associée au test de Student est inférieure à 0,05 (test de nullité du coefficient de régression linéaire).

Le $R^2$ associé au modèle est un indicateur pour quantifier le pourcentage de variabilité expliqué par le composé. Il peut ne pas être très élevé mais être significatif, c'est pourquoi on choisit de sélectionner les composés en fonction de la probabilité critique (afin de ne pas négliger un composé qui influe peu mais significativement sur le classement sensoriel).

*Coefficients:*

|  | Estimé | Erreur Std | valeur t | **Pr(>|t|)** |
|---|---|---|---|---|
| *(ordonnée à l'origine)* | *5,689e-01* | *1,112e+00* | *0,512* | *0,621* |
| **2-éthyl-1-hexanol** | **-2,295e-09** | **1,710e-08** | **-0,134** | **0,896** |

*Erreur standard résiduelle: 2,473 sur 9 degrés de liberté*
$R^2$ *Multiple: 0,001998*   $R^2$ *ajusté: -0,1089*
*Statistique F: 0,01801 sur 1 et 9 degrés de liberté, Valeur p: 0,8962*

Dans cet exemple sur le 2 éthyl-1-hexanol, la probabilité critique est supérieure à 0,05 donc il n'existe pas de lien linéaire entre le composé et le classement sensoriel établi.

*Coefficients:*

|  | Estimé | Erreur Std. | valeur t | **Pr(>|t|)** |
|---|---|---|---|---|
| *(ordonnée à l'origine)* | *-8,642e-01* | *2,729e-01* | *-3,167* | *0,0114 \** |
| **3-octène-2-one** | **5,336e-08** | **5,812e-09** | **9,181** | **7,25e-06** *\*\*\** |

*---*
*Codes de significativité:*   *0 '\*\*\*' 0.001 '\*\*' 0.01 '\*' 0.05 '.' 0.1 " 1*
*Erreur standard résiduelle: 0,7688 sur 9 degrés de liberté*
**$R^2$ Multiple: 0,9035**,   $R^2$ *ajusté: 0,8928*
*Statistique F: 84,3 sur 1 et 9 degrés de liberté, Valeur p: 7,251e-06*

Dans cet autre exemple sur le 3-octène-2-one, la probabilité critique est inférieure à 0,05 et le $R^2$ Multiple est proche de 1 (0,90). Donc, ce composé est significativement et fortement lié au classement sensoriel.

Au final, 13 composés ont été identifiés: heptanal, 3-octène-2-one, 2,4-heptadiénal, 3,5-octadiène-2-one, 2,4-nonadiénal, 2,4-décadiénal, acide hexanoïque, acide 2-éthylhexanoïque, acide heptanoïque, myristate-1, laurate-1, myristate-2, et géranyl-acétone.

On retrouve donc ici représentées les familles des aldéhydes di-insaturés, des cétones insaturés, des acides carboxyliques et des dérivés d'acides carboxyliques.

La valeur des ions spécifiques (par SPME GC/MS) de chacune de ces 13 molécules est donnée dans le tableau 4 suivant.

Tableau 4 (temps d'élution)

|  | Temps de rétention (min) | Ion spécifique (m/z) |
|---|---|---|
| 2,4-décadiénal | 27.2 | 81 |
| 2,4-heptadiénal | 19.6 | 81 |
| 2,4-nonadiénal | 24.7 | 81 |
| 3,5-octadiène-2-one | 20.2 | 95 |
| 3-octène-2-one | 17.3 | 111 |
| Heptanal | 11.2 | 70 |
| Acide 2-éthylhexanoïque | 30.0 | 88 |
| Acide heptanoïque | 30.1 | 60 |
| Acide hexanoïque | 27.7 | 60 |

(suite)

|  | Temps de rétention (min) | Ion spécifique (m/z) |
|---|---|---|
| géranyl-acétone | 28.0 | 151 |
| myristate-1 | 31.8 | 228 |
| laurate-1 | 37.2 | 112 |
| myristate-2 | 41.1 | 228 |

Le Tableau 5 suivant présente les sommes des surfaces des pics d'ions spécifiques en SPME / GC-MS de ces 13 molécules pour chacun des 10 lots de compositions de farine de microalgues (valeur relative : 100 % attribuées au lot de Réf 2).

Tableau 5.

|  | Total des surfaces des ions spécifiques des 13 molécules (%) |
|---|---|
| Lot 111 | 1,4 |
| Lot 31 | 2,2 |
| Lot 21 | 6,3 |
| Lot 23 | 4,7 |
| Lot Réf 1 | 13,3 |
| Lot 131 | 15,2 |
| Lot 24 | 51,7 |
| Lot 53 | 64,3 |
| Lot 61 | 58,4 |
| Lot Réf 2 | 100 |

Les seuils olfactifs dans l'eau, attribués à ces 13 composés sont présentés dans le tableau 6 suivant.

Tableau 6.

|  | seuil olfactif dans l'eau (ppb) |
|---|---|
| 2,4-décadiénal | 0,07 |
| 2,4-heptadiénal* | 0,1 |
| 2,4-nonadiénal | 0,01 |
| 3,5-octadiène-2-one* | 1 |
| 3-octène-2-one* | 1 |
| Heptanal | 3 |
| Acide 2-éthylhexanoïque * | 1000 |
| Acide heptanoïque | 3000 |
| Acide hexanoïque | 3000 |
| géranyl-acétone | 60 |
| myristate-1* | 1 |
| laurate-1* | 1 |
| myristate-2* | 1 |
| (*) seuil olfactif établi par la société Demanderesse | |

Le tableau 7 suivant présente la somme des valeurs d'arômes individuelles de ces 13 composés, donc la valeur d'arome totale, déterminée à partir des teneurs relatives des 13 composés et leurs seuils olfactifs (de la même manière que pour la première approche) :

$$VA\ total = \Sigma VAx\ (\text{somme des VA individuelles}),$$

avec *VAx = Concentration du composé x / seuil olfactif du composé x*) pour chacun des 10 lots de compositions de farine de microalgues (valeur de 100 % attribuée au lot Réf 2).

Tableau 7

|  | Valeur d'arôme totale (%) |
|---|---|
| Lot 111 | 0,92 |
| Lot 31 | 0,44 |
| Lot 21 | 0,52 |
| Lot 23 | 0,40 |
| Lot Réf 1 | 17,7 |
| Lot 131 | 24,33 |
| Lot 24 | 31,7 |
| Lot 53 | 57,3 |
| Lot 61 | 65,6 |
| Lot Réf 2 | 100 |

Cette analyse par GC/MS permet également de considérer l'échelle de valeurs suivantes :
lot 111 > lot 31 > lot 21 > lot 23 > Lot référence 1 > lot 131 > lot 24 > lot 53 > lot 61 > lot référence 2.
Il y a bien une correspondance parfaite entre l'échelle d'analyse sensorielle en formulation alimentaire et celle des valeurs d'arômes des 13 molécules.
Les compositions de farine de microalgues conformes à l'invention, notamment celles présentant un profil sensoriel optimisé, peuvent donc être définies par leur teneur relative en 13 composés organiques volatils particuliers.

**Description des Figures**

**FIGURE 1 :** Représentation graphique des différents lots (nuage de points) de l'ACP
**FIGURE 2 :** Cercle de corrélation de l'ACP représentant les profils aromatiques des différents lots
**FIGURE 3** : Profils chromatographiques représentant les absorbances relatives en fonction du temps pour les différents lots testés. La flèche représente le classement sensoriel de bon (en haut) à mauvais (en bas).
**FIGURE 4 :** Valeurs d'arômes des lots testés en considérant le nonanal et le t,t-2,4-décadiénal.

**Revendications**

**1.** Méthode pour déterminer la qualité organoleptique d'une composition de farine de microalgues comprenant la détermination de la teneur totale en 13 composés organiques volatils, les 13 composés organiques volatils étant l'heptanal, le 3-octène-2-one, le 2,4-heptadiénal, la 3,5-octadiène-2-one, le 2,4-nonadiénal, le 2,4-décadiénal, l'acide hexanoïque, l'acide 2-éthylhexanoïque, l'acide heptanoïque, le myristate-1, le laurate-1, le myristate-2 et la géranyl-acétone, la teneur totale en 13 composés organiques volatils étant comparée à celle de composition(s) de farine de microalgues de référence pour laquelle ou lesquelles les qualités organoleptiques sont définies, notamment comme inacceptable ou acceptable.

**2.** Méthode selon la revendication 1, dans laquelle la teneur totale en 13 composés organiques volatils est déterminée par SPME / GC, de préférence par SPME / GC-MS.

3. Méthode selon l'une quelconque des revendications 1-2, **caractérisée en ce que** la teneur totale en 13 composés organiques volatils est déterminée par la surface totale des pics de chromatographie après SPME / GC correspondant aux 13 composés organiques volatils.

4. Méthode selon l'une quelconque des revendications 1-3, **caractérisée en ce que** la surface totale des pics de chromatographie correspondant aux 13 composés organiques volatils est comparée à celle de composition(s) de farine de microalgues de référence pour laquelle ou lesquelles les qualités organoleptiques sont définies, notamment comme inacceptable ou acceptable.

5. Composition de farine de microalgues présentant une qualité organoleptique optimisée, **caractérisée en ce que** la surface totale des pics de chromatographie après SPME / GC correspondant à 13 composés organiques volatils, les 13 composés organiques volatils étant l'heptanal, le 3-octène-2-one, le 2,4-heptadiénal, la 3,5-octadiène-2-one, le 2,4-nonadiénal, le 2,4-décadiénal, l'acide hexanoïque, l'acide 2-éthylhexanoïque, l'acide heptanoïque, le myristate-1, le laurate-1, le myristate-2 et la géranyl-acétone, est comprise entre 1 et 25 % par rapport à celle d'une composition de farine de microalgues de référence de qualité organoleptique inacceptable.

**Patentansprüche**

1. Verfahren zur Bestimmung der organoleptischen Qualität einer Zusammensetzung von Mikroalgenmehl, umfassend die Bestimmung des Gesamtgehaltes von 13 flüchtigen organischen Verbindungen, wobei die 13 flüchtigen organischen Verbindungen Heptanal, 3-Octen-2-on, 2,4-Heptadienal, 3,5-Octadien-2-on, 2,4-Nonadienal, 2,4-Decadienal, Hexansäure, 2-Ethylhexansäure, Heptansäure, Myristat-1, Laurat-1, Myristat-2 und Geranylaceton sind, wobei der Gesamtgehalt von 13 flüchtigen organischen Verbindungen mit dem Gesamtgehalt einer Referenz-Zusammensetzung oder Referenz-Zusammensetzungen von Mikrolagenmehl verglichen wird, für die die organoleptischen Qualitäten besonders als inakzeptabel oder akzeptabel definiert sind.

2. Verfahren gemäß Anspruch 1, wobei der Gesamtgehalt von 13 flüchtigen organischen Verbindungen mithilfe SPME / GC, vorzugsweise mithilfe SPME / GC-MS bestimmt wird.

3. Verfahren gemäß einem der Ansprüche 1 bis 2, **dadurch gekennzeichnet, dass** der Gesamtgehalt von 13 flüchtigen organischen Verbindungen mithilfe der Gesamtfläche der Chromatographie-Peaks nach SPME / GC bestimmt wird, die den 13 flüchtigen organischen Verbindungen entsprechen.

4. Verfahren gemäß einem der Ansprüche 1 bis 3, **dadurch gekennzeichnet, dass** die Gesamtfläche der Chromatographie-Peaks, die den 13 flüchtigen organischen Verbindungen entsprechen, mithilfe der Gesamtfläche einer Referenz-Zusammensetzung oder Referenz-Zusammensetzungen von Mikroalgenmehl verglichen wird, für die die organoleptischen Qualitäten besonders als inakzeptabel oder akzeptabel definiert sind.

5. Zusammensetzung von Mikroalgenmehl, die eine optimierte organoleptische Qualität aufweist, **dadurch gekennzeichnet, dass** die Gesamtfläche der Chromatographie-Peaks nach SPME / GC, die den 13 flüchtigen organischen Verbindungen entsprechen, wobei die 13 flüchtigen organischen Verbindungen Heptanal, 3-Octen-2-on, 2,4-Heptadienal, 3,5-Octadien-2-on, 2,4-Nonadienal, 2,4-Decadienal, Hexansäure, 2-Ethylhexansäure, Heptansäure, Myristat-1, Laurat-1, Myristat-2 und Geranylaceton sind, zwischen 1 und 25% bezogen auf die Gesamtfläche einer Referenz-Zusammensetzung von Mikroalgenmehl mit inakzeptabler organoleptischer Qualität beträgt.

**Claims**

1. A method for determining the organoleptic quality of a microalgal flour composition, comprising determining the total content of 13 volatile organic compounds, the 13 volatile organic compounds being heptanal, 3-octen-2-one, 2,4-heptadienal, 3,5-octadien-2-one, 2,4-nonadienal, 2,4-decadienal, hexanoic acid, 2-ethylhexanoic acid, heptanoic acid, myristate-1, laurate-1, myristate-2 and geranyl acetone, the total content of 13 volatile organic compounds being compared to that of a reference microalgal flour composition or compositions for which the organoleptic qualities are defined, in particular as unacceptable or acceptable.

2. The method as claimed in claim 1, wherein the total content of 13 volatile organic compounds is determined by SPME / GC, preferably by SPME / GC-MS.

3. The method as claimed in any one of claims 1-2, **characterized in that** the total content of 13 volatile organic compounds is determined by the total surface area of the chromatography peaks after SPME / GC corresponding to the 13 volatile organic compounds.

4. The method as claimed in any one of claims 1-3, **characterized in that** the total surface area of the chromatography peaks corresponding to the 13 volatile organic compounds is compared to that of a reference microalgal flour composition or compositions for which the organoleptic qualities are defined, in particular as unacceptable or acceptable.

5. A microalgal flour composition having an optimized organoleptic quality, **characterized in that** its total content of 13 volatile organic compounds, the 13 volatile organic compounds being heptanal, 3-octen-2-one, 2,4-heptadienal, 3,5-octadien-2-one, 2,4-nonadienal, 2,4-decadienal, hexanoic acid, 2-ethylhexanoic acid, heptanoic acid, myristate-1, laurate-1, myristate-2 and geranyl acetone, is between 1% and 25% relative to that of a reference microalgal flour composition of unacceptable organoleptic quality.

**FIGURE 1**

**FIGURE 2**

Temps (min)

**FIGURE 3**

**FIGURE 4**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010120923 A
- WO 2010045368 A
- WO 201012093 A

**Littérature non-brevet citée dans la description**

- **CALLEJON et al.** *Analytica Chimia Acta,* 2010, vol. 660, 68-75